# EUROPÄISCHE PATENTSCHRIFT

(11) **EP 2 662 150 B1**
(45) Veröffentlichungstag und Bekanntmachung des Hinweises auf die Patenterteilung: **14.11.2018**
(21) Anmeldenummer: 13166177.9
(22) Anmeldetag: 02.05.2013
(51) Int. Cl.: A61C 5/60

(54) **Mischvorrichtung für Mehrkomponentensysteme**
Mixing device for multi-component systems
Dispositif de mélange pour systèmes multicomposants

(30) Priorität: 07.05.2012 DE 102012008815
(43) Veröffentlichungstag der Anmeldung: 13.11.2013
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- WO-A1-2011/083095
- DE-A1-102010 019 217
- DE-C2- 4 412 261

## Beschreibung

Die Erfindung betrifft Mischvorrichtungen zum Herstellen eines Mehrkomponentengemischs, insbesondere für medizinische Anwendungen, das zumindest eine erste Komponente und eine zweite Komponente umfasst, wobei die erste Komponente ein fluide Masse, insbesondere eine pastöse Masse ist, wobei die Mischvorrichtung ein Gehäuse mit wenigstens einer ersten Öffnung und einen Hohlkörper umfasst, wobei der Hohlkörper einen Innenraum umfasst, der die zweite Komponente beinhaltet und die Mischvorrichtung einen Förderkolben zum Austreiben der zweiten Komponente aus dem Innenraum des Hohlkörpers umfasst.

Die Erfindung betrifft ferner ein Kartuschensystem mit einer solchen Mischvorrichtung und ein Applikator mit einem solchen Kartuschensystem. Schließlich betrifft die Erfindung auch noch ein Verfahren zum Herstellen eines Mehrkomponentengemischs, insbesondere mit einer solchen Mischvorrichtung.

Mischvorrichtungen zum Mischen und gegebenenfalls auch zum Applizieren eines Mischguts können aus mehreren Einzelteilen bestehen und sollen gute Durchmischung der beiden Komponenten in den gewünschten Mengenverhältnissen gewährleisten.

Grundsätzlich sind reaktive, pastenförmige Zwei-Komponenten-Systeme in der Technik bei radikalisch härtenden Olefinen, Epoxid-Harzen und Silikonen weit verbreitet und werden als Klebstoffe und Dichtungsmaterialien in großem Umfang hergestellt und in der Industrie, Handwerk und im Heimwerkerbereich eingesetzt. Daneben sind reaktive, pastenförmige Zwei-Komponenten-Systeme im Bereich der Dentaltechnik üblich. Reaktive pastöse Zwei- oder Mehrkomponentensyteme müssen nach ihrer Herstellung bis zur Applikation getrennt aufbewahrt werden, um vorzeitige, unbeabsichtigte Reaktionen der Komponenten zu verhindern. Kartuschensysteme für die Applikation von pastösen Zwei- oder Mehrkomponentensystemen sind seit langem bekannt. Beispielhaft seien dafür die Dokumente CH 669 164 A5, EP 0 607 102A1, EP 0 236 129 A2, DE 3 440 893 A1, US 4,690,306 A, US 2009/062808 A1, EP 0 787 535 A1, WO 2006/005 206 A1, EP 0 693 437 A1, EP 0 294 672 A, EP 0 261 466 A1 und EP 2 008 707 A1 genannt. Nach Befüllen der Kartuschen mit reaktiven Pasten müssen diese sicher bis zur Applikation verschlossen bleiben. Die Vermischung der pastösen Zwei- oder Mehrkomponentensysteme erfolgt unmittelbar bei der Applikation üblicherweise mit Hilfe von statischen Mischern. Exemplarisch seien dafür die Dokumente GB 1,188,516 A, US 2,125,245 A, US 5,968,018 A, US 4,068,830 A, US 2003/179648 A1, EP 1 799 335 A1, EP 0 664 153 A1 und EP 0 289 882 A1 angeführt. Bewegliche Kolben, die auch zum Austragen des Kartuscheninhalts dienen, dichten dabei üblicherweise die Kartuschenböden ab.

Die Rückseite der Kartuschen wird üblicherweise durch bewegliche Kolben verschlossen, die zum Austreiben der Pasten bei der Applikation bestimmt sind. Bei feuchtigkeits- und luftempfindlichen Pasten können Aluminiumkartuschen verwendet werden, die mit Kunststoffkolben verschlossen sind und über die zur Abdichtung Aluminiumzylinder, die an einer Seite geschlossen sind, eingepresst werden. Bei der Applikation der Pasten wird der einseitig geschlossene Aluminiumzylinder zusammen mit dem Kolben mit Hilfe von Auspresspistolen nach vorne in Richtung Kartuschenkopf bewegt und die Paste dabei ausgetrieben. Bei medizinischen Anwendungen kann jedoch ein Kontakt von Pasten mit Aluminiumoberflächen problematisch sein.

Gegenwärtig sind zur Lagerung von reaktiven Zwei-Komponenten-Pastensystemen so genannte side-by-side-Kartuschen und Koaxial-Kartuschen technisch üblich. Dabei werden beide für sich stabile Pasten räumlich voneinander getrennt in separaten Kartuschen gelagert und erst bei der Applikation miteinander vermischt. Für die Vermischung werden häufig statische Mischer eingesetzt. Die Aushärtungsreaktion wird erst durch die Vermischung der beiden Pasten miteinander gestartet.

In der Medizin werden seit Jahrzehnten Polymethylmethacrylat-Knochenzemente zur dauerhaften mechanischen Fixierung von Totalgelenkendoprothesen eingesetzt. Diese basieren auf Pulver-Flüssigkeits-Systemen. Es wurden in jüngster Zeit auch Polymethylmethacrylat-Knochenzemente vorgeschlagen, die auf der Verwendung von Zementpasten beruhen (DE 10 2008 030 312 A1, DE 10 2007 052 116 A1). Dabei wurden auch pastenförmige Zwei-Komponenten-Polymethylmethacrylat-Knochenzement-Systeme vorgeschlagen (DE 10 2007 050 762 A1).

Bei der Anwendung von Knochenzementen zur Fixierung von Totalgelenkendoprothesen muss immer berücksichtigt werden, dass bei diesen Operationen das OP-Personal unter zeitlichem Druck steht. Bei medizinischen Anwendungen von Kartuschensystemen für die Applikation von pastenförmigen Polymethylmethacrylat-Knochenzementen sollten diese daher grundsätzlich so gestaltet sein, dass sie weitgehend resistent gegenüber Anwenderfehlern sind und auch in Stresssituationen schnell und sicher bedient werden können.

Radikalisch aushärtende Zwei-Komponentensysteme sind grundsätzlich so aufgebaut, dass in jeweils einer Paste ein radikalischer Initiator und in der anderen Paste mindestens ein Beschleuniger enthalten ist. Man benötigt im Allgemeinen nur sehr geringe Mengen an Beschleuniger, um durch Reaktion des Beschleunigers mit dem Initiator Radikale zur Initiierung der radikalischen Polymerisation zu erzeugen. Um gleiche Mischungsverhältnisse zu erreichen haben die bekannten side-by-side Kartuschen in etwa gleiche Innendurchmesser, da ein gleichmäßiger Austrag bei einer schmalen Kartusche nicht gewährleistet ist. Das bedeutet, es sind nur geringe Mengen Beschleuniger in einem recht großen Volumen an Paste verteilt. Die Paste dient nur als Träger für den Beschleuniger.

Die zwei Pasten beziehungsweise Komponenten für das Mehrkomponentengemisch, eine mit dem Initiator und die zweite mit dem Beschleuniger, sind üblicherweise getrennt in unterschiedlichen Kartuschen gelagert. Aus diesen Kartuschen werden die Pasten durch darin angebrachte Förderkolben in Richtung Kartuschenkopf gedrückt. Es kommt dabei darauf an, dass die Pasten in dem vorbestimmten Volumenverhältnis aus den Kartuschen in statische Mischer gepresst werden, die jeweils am Kartuschenkopf angebracht sind. In den statischen Mischern vermischen sich die Pasten (Komponenten) und die Aushärtungsreaktion setzt ein. Für die Qualität des ausgehärteten Pastenmaterials ist es wesentlich, dass das Volumenverhältnis der Pasten zueinander eingehalten wird. Am häufigsten liegt das Volumenverhältnis der Pasten im Bereich 1:1 bis 1:10. Das Volumenverhältnis wird einerseits durch die Dimensionierung der Kartuschen gewährleistet und andererseits durch den synchronen Vortrieb der Förderkolben in den Kartuschen. Technisch wird dieser synchrone Vortrieb dadurch erreicht, dass die Förderkolben miteinander verbunden sind. Bei den meisten Zwei-Komponenten-Systemen sind die Förderkolben mit einer Zahnstange verbunden, die entweder durch Handkraft über Bedienhebel oder durch Motoren in Richtung des Kartuschenkopfs bewegt wird.

Daneben ist es auch möglich, Kartuschen mit Druckluft auszupressen. Dabei befindet sich in dem Applikator ein Förderkolben, der an der Außenseite zwei Stößel besitzt. Der Förderkolben wird beim Auspressen mit Druckluft beaufschlagt, wodurch sich dieser in Richtung Kartuschenkopf bewegt. Weil beide Stößel mit dem Förderkolben verbunden sind, können sich diese nur synchron nach vorne in Richtung Kartuschenkopf bewegen.

Die Synchronisierung des Austrags ist deshalb wichtig, weil die Pasten üblicherweise nicht die exakt gleiche Viskosität besitzen. Deshalb würde eine einfache Druckbeaufschlagung mit Druckluft auf nicht synchronisierte Förderkolben dazu führen, dass bei der niedrig viskoseren Paste der Förderkolben schneller in Richtung Kartuschenkopf gedrückt würde als bei der im Vergleich höher viskosen Paste. Dadurch würde das vorgegebene Volumenverhältnis verändert. Das Ergebnis wäre ein nicht optimal aushärtendes Pastenmaterial.

Die direkte Beaufschlagung der Förderkolben mit komprimierten Gas aus Druckluftleitungen oder auch aus konventionellen Druckgaspatronen, wie zum Beispiel Kohlendioxid-Patronen, hätte die Vorteile, dass die Förderkolben beziehungsweise der Förderkolben mit großen Kräfte bedient werden könnte, wodurch auch hoch viskose Pasten ausgepresst werden könnten und der Auspressdruck und damit die Auspressgeschwindigkeit durch einfache Ventile steuerbar wäre, ohne dass mechanische Vorrichtungen, wie Zahnstangen oder Getriebe notwendig wären. Applikatoren mit Druckgaspatronen sind aus den Dokumenten DE 10 2010 019 222 A1 und DE 10 2010 019 224 B3 bekannt.

Eine gattungsgemäße Mischvorrichtung zum Mischen und Applizieren eines Mischguts ist aus der DE 10 2010 019 217 A1 bekannt. Die Mischvorrichtung umfasst zumindest zwei Kartuschen und einen Mischraum, der mit den Kartuschen durch jeweils eine Öffnung verbunden ist. In den Kartuschen sind Förderkolben zum Austreiben der Ausgangskomponenten angeordnet. Die Förderkolben werden durch Beaufschlagung mit einem Druck in die Kartuschen hereingedrückt und dadurch die Komponenten aus den Kartuschen herausgedrückt und miteinander vermischt. Eine Mischvorrichtung, sowie ein Verfahren mit den Merkmalen des Oberbegriffs der unabhängigen Ansprüche 1 und 17 wird vom Dokument WO95/27558 A offenbart.

Nachteilig an allen bekannten Systemen ist, dass die Systeme zwei Kartuschen haben und daher nicht ohne weiteres mit Druckgas betrieben werden können. Zudem benötigt die Kartusche mit dem Beschleuniger ein unnötig großes Volumen, da sich ansonsten kein gleichmäßiger Austrag und damit keine gleichmäßigen Mischungen erzeugen lassen.

Aufgabe der Erfindung ist es, diese und weitere nicht genannte Nachteile zu überwinden. Insbesondere soll eine Mischvorrichtung bereitgestellt werden, die auch mit Gasdruck problemlos betrieben werden kann. Eine Mischvorrichtung mit einem geringeren Platzbedarf wäre zudem vorteilhaft. Die Mischvorrichtung soll auch einfach in der Bedienung sein und möglichst störunanfällig einzusetzen sein.

Aufgabe der Erfindung ist es ferner, ein Kartuschensystem beziehungsweise eine Mischvorrichtung zum Vermischen und Austragen von Polymethylmethacrylat-Knochenzementpaste zu entwickeln, welche die Verwendung von nur einer Polymethylmethacrylat-Knochenzementpaste ermöglicht, die jedoch trotzdem mit einem aus mindestens einem Initiator und einem Beschleuniger aufgebauten RedoxinitiatorSystem, zur Aushärtung gebracht werden kann. Es soll dazu eine möglichst kleinvolumige Vorrichtung bereitgestellt werden, die beim Herauspressen der Zementpaste geringe, jedoch definierte Mengen an Beschleuniger oder Initiator so abgibt, dass die gesamte aus der Kartusche herausgepresste Zementpaste mit immer der gleichen Menge an Beschleuniger oder Initiator vermischt wird.

Diese Aufgaben werden gelöst durch eine Mischvorrichtung mit den Merkmalen des Anspruchs 1 zum Herstellen eines Mehrkomponentengemischs, insbesondere für medizinische Anwendungen, das zumindest eine erste Komponente und eine zweite Komponente umfasst, wobei die erste Komponente ein fluide Masse, insbesondere eine pastöse Masse ist, wobei die Mischvorrichtung ein Gehäuse mit wenigstens einer ersten Öffnung und einen Hohlkörper umfasst, wobei der Hohlkörper einen Innenraum umfasst, der die zweite Komponente beinhaltet und die Mischvorrichtung einen Förderkolben zum Austreiben der zweiten Komponente aus dem Innenraum des Hohlkörpers umfasst, wobei der Hohlkörper ein Gewinde aufweist, der Förderkolben ein Gegengewinde umfasst, das in das Gewinde des Hohlkörpers greift, und die Mischvorrichtung ein Vortriebselement umfasst, das innerhalb des Gehäuses angeordnet ist, wobei das Vortriebselement eine Strömung der ersten Komponente durch das Gehäuse in eine Drehbewegung umsetzt, wobei die Drehbewegung des Vortriebselements den Förderkolben in den Innenraum des Hohlkörpers schraubt und dadurch die zweite Komponente aus dem Hohlraum in den Fluidstrom der ersten Komponente pressbar ist.

Die erste Komponente ist erfindungsgemäß bevorzugt eine pastöse Masse, wobei auch die zweite Komponente bevorzugt eine pastöse Masse sein kann. Beide Komponenten sind besonders bevorzugt Komponenten medizinischer Zemente.

Dabei kann vorgesehen sein, dass das Gewinde des Hohlkörpers ein Innengewinde ist und das Gegengewinde des Förderkolbens ein Außengewinde ist, das in das Innengewinde des Hohlkörpers greift, wobei vorzugsweise die Innenwand des Innenraums durch das Innengewinde gebildet wird.

Durch diesen Aufbau ist der funktionelle Innenaufbau der Mischvorrichtung kompakter gestaltbar und damit die gesamte Mischvorrichtung in den äußeren Abmessungen kleiner gestaltbar. Zudem können die im Inneren des Hohlkörpers angeordneten Gewinde nicht von Füllstoffen der ersten Komponente beeinträchtigt werden.

Gemäß einer bevorzugten Weiterbildung der Erfindung kann vorgesehen sein, dass der Hohlkörper, vorzugsweise über Stege fest mit dem Gehäuse verbunden ist und das Vortriebselement fest mit dem Förderkolben verbunden ist oder der Förderkolben, vorzugsweise über Stege fest mit dem Gehäuse verbunden ist und das Vortriebselement fest mit dem Hohlkörper verbunden ist.

In beiden Fällen trägt das Gehäuse einen Teil des Innenaufbaus, so dass sich der Förderkolben in den Hohlraum des Hohlkörpers schrauben kann.

Ferner kann vorgesehen sein, dass der Förderkolben gegen den Hohlkörper drehbar gelagert ist und in Längsrichtung des Innenraums des Hohlkörpers schraubbar gelagert ist.

Durch diese Maßnahme wird erreicht, dass ein einfacher Aufbau für den Innenaufbau der erfindungsgemäßen Mischvorrichtung gewählt werden kann.

Des Weiteren kann vorgesehen sein, dass der Innenraum des Hohlkörpers zwei Öffnungen aufweist, wobei eine Öffnung durch den Förderkolben verschlossen ist und bevorzugt die andere Öffnung in Richtung des Fluidstroms der ersten Komponente geöffnet ist, wobei besonders bevorzugt der Innenraum des Hohlkörpers im Wesentlichen zylindrisch geformt ist und die beiden Öffnungen an den Grundflächen des zylindrischen Innenraums angeordnet sind.

Dieser Aufbau bewirkt, dass der Inhalt des Hohlkörpers, also die zweite Komponente besonders leicht aus dem Hohlraum herausdrückbar ist.

Gemäß einer besonders bevorzugten Ausführungsform der Mischvorrichtung kann vorgesehen sein, dass das Vortriebselement eine Förderschraube ist oder zumindest zwei Flügel und/oder eine Flügelscheibe umfasst, wobei die Flügel und/oder die Flügelscheibe zumindest eine gegen den Fluidstrom der ersten Komponente geneigte Fläche umfasst oder umfassen oder vorzugsweise mehrere gegen den Fluidstrom der ersten Komponente geneigte Flächen umfasst oder umfassen.

Solche Flügel, Propeller oder Turbinenschaufeln sind besonders gut zum Antrieb des Förderkolbens oder des Hohlkörpers geeignet.

Dabei kann vorgesehen sein, dass die geneigte Fläche oder die geneigten Flächen der Flügel und/oder Flügelscheibe zwischen 5° und 85° gegen den Fluidstrom der ersten Komponente geneigt ist oder sind, vorzugsweise zwischen 30° und 60°, besonders bevorzugt um 45°.

Eine Weiterbildung der erfindungsgemäßen Mischvorrichtung sieht vor, dass an dem Vortriebselement bezogen auf die Drehachse des Vortriebselements außen liegend Kontaktpunkte vorgesehen sind, über die das Vortriebselement im Inneren des Gehäuses führbar sind, wobei das Gehäuse zumindest in diesem Bereich innen zylindrisch ist.

Durch die Kontaktpunkte wird das Vortriebselement in dem Gehäuse geführt, ohne dass es dabei blockieren kann.

Bevorzugt kann auch vorgesehen sein, dass das Mehrkomponentengemisch ein Zweikomponentengemisch ist, insbesondere ein aushärtender medizinischer Zement, wobei eine Komponente einen Beschleuniger umfasst und die andere Komponente einen Initiator umfasst, wobei bevorzugt die erste Komponente Methacrylat-Monomere und darin gelöste Polymere umfasst und besonders bevorzugt eine Polymethylmethacrylat-Knochenzementpaste ist und die zweite Komponente einen den Initiator, insbesondere ein radikalisches Initiatorsystem umfasst.

Für diese Mehrkomponentengemische, insbesondere für die medizinischen Zemente gibt es besonders geeignete Ausgangskomponenten. Zudem ist der kompakte Aufbau bei manueller Anwendung besonders vorteilhaft.

Ferner kann vorgesehen sein, dass an das Gehäuse eine Kartusche anschließbar ist oder befestigt ist, vorzugsweise an einer zweiten Öffnung des Gehäuses, wobei die Kartusche die erste Komponente enthält und die Kartusche einen Kolben zum Austreiben der ersten Komponente aus der Kartusche und zum Erzeugen des Fluidstroms der ersten Komponente durch das Gehäuse umfasst, wobei bevorzugt die Kartusche über ein Befestigungsmittel mit dem Gehäuse verbindbar oder verbunden ist, besonders bevorzugt über ein Gewinde, einen Bajonett-Verschluss oder eine Rastung.

Wenn die Kartusche fest mit dem Gehäuse verbunden oder verbindbar ist, kann der Fluidstrom der ersten Komponente auch durch starken mechanischen Druck auf den Kolben erzeugt werden.

Auch kann vorgesehen sein, dass an das Gehäuse ein Austragsrohr als Fortsetzung der wenigstens einen ersten Öffnung anschließbar ist oder befestigt ist, so dass das Mehrkomponentengemisch über das Austragsrohr austragbar ist, wobei bevorzugt im Inneren des Austragsrohrs ein statischer Mischer zum Durchmischen des Mehrkomponentengemischs angeordnet ist.

Das Austragsrohr erhöht die Anwenderfreundlichkeit des Aufbaus. Durch den statischen Mischer wird die Mischwirkung der Mischvorrichtung verbessert.

Gemäß einer Weiterbildung der Erfindung kann vorgesehen sein, dass die zweite Komponente ein Feststoff ist, der in dem Innenraum des Hohlkörpers steckt und der sich in dem Fluidstrom der ersten Komponente löst.

Feststoffe als zweite Komponenten lassen sich besonders einfach in den Hohlraum stecken. Zudem können diese auch nicht vorher auslaufen, so dass dann auf ein Ventil oder eine Schutzfolie auf der Öffnung des Hohlkörpers verzichtet werden kann, durch die die zweite Komponente ausgetragen werden soll.

Es kann des Weiteren vorgesehen sein, dass der Hohlkörper zumindest bereichsweise innerhalb des Gehäuses angeordnet ist, vorzugsweise der Hohlkörper und der Förderkolben vollständig im Inneren des Gehäuses angeordnet sind, so dass beide von dem Fluidstrom der ersten Komponente umfließbar sind.

Hierdurch wird ein besonders einfacher und kompakter Aufbau erreicht. Der Hohlkörper kann so zudem von außen nicht beschädigt werden und ist für den Anwender nicht sichtbar.

Eine weitere bevorzugte erfindungsgemäße Mischvorrichtung zeichnet sich dadurch aus, dass beim Einschrauben des Förderkolbens in den Innenraum des Hohlkörpers sich das Volumen des Innenraums des Hohlkörpers proportional zu der Umdrehung des Vortriebselements und damit proportional zu der Umdrehung des Förderkolbens gegen den Hohlkörper vermindert und die Umdrehungsgeschwindigkeit des Förderkolbens proportional zum Volumenstrom der ersten Komponente durch das Vortriebselement ist.

Die zweite Komponente wird so in definierten Mengen in die erste Komponente gemischt. Es wird stets das richtige Mischungsverhältnis erzwungen.

Es ist erfindungsgemäß besonders bevorzugt, wenn die zweite Komponente eine thixotrope Flüssigkeit ist.

Gemäß einer weiteren besonders bevorzugten Ausgestaltung der Erfindung kann vorgesehen sein, dass das Gegengewinde des Förderkolbens in der Ausgangsposition im Wesentlichen oder vorzugsweise vollständig von dem Gewinde des Hohlkörpers bedeckt ist. Insbesondere dann, wenn das Gegengewinde ein Innengewinde ist und das Gewinde des Hohlkörpers ein Außengewinde, führt dies dazu, dass Substanzen wie Röntgenopaker (ZrO₂-Partikel) das Gewinde während des Mischens nicht blockieren. Es ist besonders vorteilhaft, wenn die Öffnung, durch die die zweite Komponente aus dem Hohlkörper gedrückt wird, in Richtung des Fluidstroms der ersten Komponente ausgerichtet ist. Durch diese Maßnahme kann eine besonders gleichmäßige und leichtgängige Vermischung der Komponenten erreichet werden.

An der Öffnung, durch die die zweite Komponente aus dem Hohlkörper gedrückt wird, kann erfindungsgemäß besonders bevorzugt ein Ventil, insbesondere ein Lippenventil oder eine Schutzmembran vorgesehen sein. Durch diese Maßnahmen kann eine Verunreinigung der zweiten Komponente verhindert werden. Zudem kann so einer unerwünschten Austrocknung des Inhalts des Hohlkörpers entgegengewirkt werden.

Die Aufgaben der Erfindung werden auch gelöst durch ein Kartuschensystem umfassend eine solche Mischvorrichtung, eine Kartusche, die die erste Komponente beinhaltet und die einen Kolben zum Ausdrücken der ersten Komponente in die Mischvorrichtung umfasst.

Des Weiteren werden die Aufgaben der Erfindung gelöst durch einen Applikator umfassend ein solches Kartuschensystem, ein bedienbares Ventil und eine Druckgaspatrone, insbesondere eine CO2-Patrone zur Beaufschlagung des Kolbens der Kartusche mit einem Gasdruck.

Die Aufgaben der Erfindung werden auch gelöst durch ein Verfahren mit den Merkmalen des Anspruchs 17 zum Herstellen eines Mehrkomponentengemischs, insbesondere mit einer solchen Mischvorrichtung, bei dem eine erste Komponente in ein Gehäuse gepresst wird und dort einen Fluidstrom erzeugt, wobei der Fluidstrom ein Vortriebselement antreibt und in Drehung versetzt und durch das sich drehende Vortriebselement ein Förderkolben in einen Innenraum eines Hohlkörpers geschraubt wird, wobei dadurch eine zweite Komponente aus dem Inneren des Hohlköpers in den Fluidstrom der ersten Komponente gedrückt wird und sich dort mit der ersten Komponente mischt und das Gemisch aus dem Gehäuse heraus gedrückt wird.

Dabei kann vorgesehen sein, dass beim Einschrauben des Förderkolbens in den Innenraum des Hohlkörpers das Volumen des Innenraums des Hohlkörpers proportional zu der Umdrehung des Vortriebselements und damit des Förderkolbens gegen den Hohlkörper vermindert wird und eine Umdrehungsgeschwindigkeit des Förderkolbens proportional zum Volumenstrom der ersten Komponente durch das Vortriebselement erzeugt wird.

Bei der Verwendung von Kartuschensystemen für sterile pastöse Medizinprodukte ist es notwendig, dass nicht nur die Pasten sondern natürlich auch die Kartuschen und das sekundäre Packmittel in steriler Form dem medizinischen Anwender zur Verfügung gestellt werden. So können zum Beispiel nach aseptischer Befüllung der zuvor sterilisierten Kartuschen diese direkt in ein steriles Packmittel überführt werden. Weiterhin kann es bei bestimmten Produkten sinnvoll sein, die Oberflächen der befüllten Kartuschen zusammen mit den Packmitteln nach erfolgter Verpackung zu sterilisieren. Neben der Gammasterilisation, die bei polymerisierbaren Pastensystemen nicht angewendet werden kann, besteht die Möglichkeit mit dem Gas Ethylenoxid zu sterilisieren.

Unter dem Begriff Polymethylmethacrylat-Knochenzementpaste werden hierbei insbesondere pastenförmige Zubereitungen aus mindestens einem Methacrylat-Monomeren und einem darin gelösten Polymeren und mindestens einem Bestandteil eines radikalischen Initiatorsystems verstanden, wobei diese Pasten in einem maximal gequollen Zustand vorliegen und in diesem maximal gequollenen Zustand bis zu ihrer Verwendung gelagert werden.

Aus Mischungen von Zementpulvern mit Monomerflüssigkeiten gebildete pastenförmige Zementteige konventioneller Pulver-Flüssigkeits-Polymethylmethacrylat-Knochenzemente sind dagegen nur schwerer einsetzbar. Bei diesem pastenförmigen Zementteig erfolgt eine fortlaufende Quellung der im Zementpulver enthaltenen Polymerpartikel in der Monomerflüssigkeit, wobei gleichzeitig unter Verbrauch des Monomers eine radikalische Polymerisation des Monomers erfolgt. Dieser pastenförmige Zementteig härtet innerhalb weniger Minuten aus und ist daher nicht lagerfähig.

Der Erfindung liegt die überraschende Erkenntnis zugrunde, dass durch die Verbindung eines Hohlkörpers und eines Förderkolbens darin über ein Gewinde gelingt, einen definierten Vortrieb des Förderkolbens zu erzielen, wenn dieser durch eine Vortriebseinrichtung geschraubt wird, die durch den Volumenstrom der ersten Komponente betrieben wird. Der Vortrieb des Förderkolbens hängt dann unmittelbar mit der Stärke des Volumenstroms der ersten Komponente durch das Gehäuse zusammen, in dem die Vortriebseinrichtung angeordnet ist. Der Förderkolben kann so sehr definiert auch kleine Mengen der zweiten Komponente aus dem Hohlraum des Hohlkörpers herausdrücken und dabei in die erste Komponente mischen. Dadurch wird immer das gewünschte Mischungsverhältnis erzielt unabhängig von dem Druck, der auf den Kolben der Kartusche mit der ersten Komponente wirkt.

Durch die Erfindung gelingt es, ein gewünschtes Mischungsverhältnis herzustellen, auch wenn das Volumen der zweiten Komponente wesentlich kleiner als das der ersten Komponente ist.

Ganz besonders vorteilhaft ist die Erfindung auch dadurch, dass es mit der erfindungsgemäßen Vorrichtung gelingt, einen Gasdruck direkt auf möglichst nur einen Kolben einwirken zu lassen, ohne dass eine großvolumige Synchronisierung in Form von synchronisierten Stößeln oder anderen mechanischen Vorrichtungen notwendig wäre. Die erfindungsgemäße Mischvorrichtung ist auch deshalb besonders vorteilhaft, da es auf einen Kartuschenkopf oder in ein Austragsrohr eingebracht werden kann und daher besonders kompakt ist.

Für einen pastenförmigen Polymethylmethacrylat-Knochenzement muss mit Hilfe der Erfindung im Interesse möglichst niedriger Herstellungskosten anstelle von zwei Polymethylmethacrylat-Knochenzementpasten nur eine Polymethylmethacrylat-Knochenzementpaste hergestellt werden und diese nur eine Paste kann in nur eine einfache, preisgünstige Kartusche abgefüllt werden. Erfindungsgemäß können diese jedoch genau so sicher wie bei bekannten Zwei-Komponenten-Pastensystemen durch ein Redoxinitiatorsystem, das aus mindestens einen Initiator und mindestens einen Beschleuniger aufgebaut ist, durch Redoxinitiierung zur Polymerisation und zur Aushärtung gebracht werden.

Ein zentraler Vorteil der Erfindung besteht also darin, dass nur eine einzelne Kartusche mit nur einem Kolben durch direkte Beaufschlagung mit Druckgas ausgepresst werden muss. Damit ist es möglich, mit einem einfachen Applikator, der eine Druckgaspatrone enthält, diese Kartusche auszupressen. Solche Applikatoren mit und für Druckgaspatronen sind aus den Dokumenten DE 10 2010 019 222 A1 und DE 10 2010 019 224 B3 bekannt.

Für eine Polymethylmethacrylat-Zementpaste, die aus mindestens einem radikalisch polymerisierbaren Monomer und einem darin gelösten Polymer sowie gegebenenfalls Füllstoffen aufgebaut ist, wird davon ausgegangen, dass sie entweder einen Initiator oder auch einen Beschleuniger enthält. Diese Zementpaste kann in eine einfache Kartusche gefüllt werden, wobei die Kartusche einen Austragskolben enthält und sich am Kartuschenkopf ein Anschluss für ein Austragsrohr befindet. Dieser eine Austragskolben kann in einfacher Weise mit Druckgas beaufschlagt werden und dadurch kann die Paste in Richtung Kartuschenkopf gepresst werden.

Eine besonders einfache Umsetzung für eine erfindungsgemäße Mischvorrichtung kann dabei vorsehen, dass die Mischvorrichtung auf dem Kartuschenkopf oder in dem Austragsrohr beziehungsweise als Teil des Austragsrohrs derart angeordnet wird, dass es sich beim Herauspressen der Paste direkt im Volumenstrom der Paste befindet und ganz oder teilweise von der strömenden Paste durchspült wird. Die Mischvorrichtung kann dazu aus einem zylindrischen Hohlkörper mit Innengewinde und aus einem Zylinder mit Außengewinde als Förderkolben aufgebaut werden. In dem Hohlkörper ist der Beschleuniger oder der Initiator in einer festen, halbfesten oder flüssigen Form enthalten. Der Hohlkörper hat mindestens zwei Öffnungen. Mindestens eine Öffnung ist mit der Umgebung flüssigkeitsdurchlässig verbunden. Der Zylinder mit Außengewinde steht mit seinem Außengewinde im Eingriff zum Innengewinde des Hohlkörpers und verschließt eine Öffnung des Hohlkörpers. Der Zylinder ist relativ verdrehbar zum Hohlkörper angeordnet. Der Hohlkörper oder der Zylinder (der Förderkolben) sind am Gehäuse des Austragsrohrs oder einer separaten Wand fixiert.

Weiterhin sind Flügel am Hohlkörper oder am Zylinder so angebracht, dass diese bei Umströmung der Vorrichtung durch die Zementpaste in Richtung der Austragsöffnung des Austragsrohrs, eine relative Drehbewegung des Hohlkörpers gegenüber des Zylinders so bewirken, dass der Zylinder in den Hohlkörper durch den Eingriff seines Außengewindes in das Innengewinde des Hohlkörpers gedreht wird. Dadurch wird das Volumen des Hohlraums im Hohlkörper proportional zu den Umdrehungen des Zylinders zum Hohlkörper vermindert und der im Hohlraum enthaltene Beschleuniger oder Initiator proportional zu den Umdrehungen und damit proportional zum ausgepressten Volumen der Zementpaste in die strömende Zementpaste gedrückt. Durch den im Austragsrohr nachfolgend in Richtung der Austragsöffnung angeordneten statischen Mischer wird der Beschleuniger oder auch der Initiator dann mit der strömenden Paste homogen vermischt.

Die Aufgabe der Erfindung wird also auch gelöst durch ein Kartuschensystem mit einer Mischvorrichtung für Polymethylmethacrylat-Knochenzementpaste, umfassend eine Kartusche mit Austragskolben, mit Austragsrohr und im Austragsrohr angeordneten statischen Mischer, welches dadurch charakterisiert ist, dass
a) eine Vorrichtung vor dem Kartuschenkopf oder in dem Austragsrohr so angeordnet wird, dass sich diese beim Herauspressen der Polymethylmethacrylat-Knochenzementpaste direkt im Volumenstrom der Polymethylmethacrylat-Knochenzementpaste befindet und ganz oder teilweise von der strömenden Polymethylmethacrylat-Knochenzementpaste umströmt wird,
b) die Vorrichtung aus einem zylindrischen Hohlkörper mit Innengewinde und einem Zylinder als Förderkolben umfassend ein Außengewinde aufgebaut ist,
c) in dem Hohlkörper der Beschleuniger oder der Initiator in einer festen, halbfesten oder flüssigen Form enthalten ist,
d) der Hohlkörper mindestens zwei Öffnungen besitzt.
e) mindestens eine Öffnung des Hohlkörpers den Hohlraum des Hohlkörpers flüssigkeitsdurchlässig mit der Umgebung verbindet,
f) der Zylinder mit Außengewinde mit seinem Außengewinde im Eingriff zum Innengewinde des Hohlkörpers steht und eine Öffnung des Hohlkörpers verschließt,
g) der Zylinder relativ drehbar zum Hohlkörper angeordnet ist, wobei der Hohlkörper oder der Zylinder am Austragsrohr oder einer Wand fixiert ist, und
h) dass Flügel am Hohlkörper oder am Zylinder so angebracht sind, dass diese bei Umströmung der Vorrichtung durch die Zementpaste in Richtung der Austragsöffnung des Austragsrohrs, eine relative Drehbewegung des Hohlkörpers gegenüber des Zylinders bewirken, dass der Zylinder in den Hohlkörper durch den Eingriff seines Außengewindes in das Innengewinde des Hohlkörpers gedreht wird, wobei das Volumen des Hohlraums im Hohlkörper proportional zu den Umdrehungen des Zylinders zum Hohlkörper vermindert wird und der im Hohlraum enthaltene Beschleuniger oder Initiator proportional zu den Umdrehungen und damit proportional zum ausgepressten Volumen der Zementpaste in die strömende Zementpaste gedrückt wird.

Die Flügel bilden dabei die Vortriebseinrichtung der Mischvorrichtung. Als Gehäuse wird hier das existierende Gehäuse des Austragsrohrs verwendet, beziehungsweise das Gehäuse der Mischvorrichtung wird in einem mit dem Gehäuse des Austragsrohrs aufgebaut. Ebenso ist es möglich das Gehäuse der Kartusche für die erste Komponente als Gehäuse der Mischvorrichtung zu nutzen, beziehungsweise das Gehäuse der Mischvorrichtung in einem mit dem Gehäuse der Kartusche aufzubauen.

Vor der Verwendung der Polymethylmethacrylat-Knochenzementpaste befindet sich diese in der verschlossenen Kartusche. Der Verschluss kann durch Stopfen oder auch durch geeignete Ventile verwirklicht sein.

Der mit Beschleuniger oder Initiator befüllte Hohlkörper kann vor der Anwendung entweder bereits im separaten Austragsrohr eingebaut sein oder er kann unmittelbar vor der Verwendung der Polymethylmethacrylat-Knochenzementpaste in das Austragsrohr eingesetzt werden.

Unmittelbar vor der Verwendung wird die Kartusche geöffnet und das Austragsrohr mit dem darin enthaltenden statischen Mischer und der Vorrichtung mit der geöffneten Kartusche verbunden. Die Verbindung kann dabei durch Verschraubung oder auch durch Verrastung erfolgen. Nach der Verbindung der geöffneten Kartusche mit dem Austragsrohr und der darin enthaltenen Vorrichtung und dem statischen Mischer ist das Kartuschensystem einsatzbereit.

Als Beschleuniger kommen bei Raumtemperatur flüssige, elektronenreiche aromatische Amine, wie N,N-Dimethyl-anilin und N,N-Dimethyl-p-toluidin, in Betracht. Diese können durch Verdicker in eine pastenförmige Konsistenz überführt werden, wodurch diese aus dem Hohlraum der Vorrichtung vor der Verwendung des Zements nicht herauslaufen können. Das aromatische Amin N,N-Bis-hydroxyethyl-p-toluidin kann erfindungsgemäß besonders bevorzugt eingesetzt werden, da es bei Raumtemperatur eine pastenförmige Konsistenz hat und leicht plastisch verformbar und auspressbar ist. Weiterhin löst es sich rasch in Methylmethacrylat und kann daher problemlos mit Knochenzementpaste vermischt werden.

Daneben sind auch andere Beschleuniger oder Beschleuniger-Kombinationen möglich. So sind Gemische aus flüssigen oder zähflüssigen, organischen Ammoniumchloriden, wie zum Beispiel Trioctylmethylammoniumchlorid (Aliquat 336), und darin suspendierten oder gelösten Schwermetallsalzen, wie Kupfer(II)methacrylat, geeignet. Besonders vorteilhaft ist die Kombination eines zähflüssigen Ammoniumchlorids mit einem ebenfalls flüssigen Schwermetallsalz. Als bei Raumtemperatur flüssige Schwermetallsalze kommen insbesondere 2-Ethylhexanoate von Schwermetallen in Betracht, wobei besonders Kupfer(II)-2-ethylhexanoat besonders gut geeignet ist.

Daneben sind auch Schwermetallsalze ohne Anwesenheit von Ammoniumchloriden als Beschleuniger möglich. Weiterhin sind als Beschleuniger Reduktionsmittel geeignet. Beispielhaft sind dafür Thioharnstoff und substituierte Thioharnstoffe.

Als Initiatoren sind organische Peroxide, wie Diacylperoxide, Dialkylperoxide und Hydroperoxide, geeignet. Daneben kommen auch substituierte Barbiturate als Initiatoren in Betracht. Beispielhaft sind dafür 1-Cyclohexyl-5-ethyl-barbiturat und 5-Butyl-barbiturat.

Erfindungsgemäß kann vorgesehen sein, dass der Hohlkörper im Austragsrohr oder in einem auf dem Kartuschenkopf befestigten Zylinder über Stege fixiert ist, wobei eine Öffnung des Hohlkörpers in Richtung der Austragsöffnung des Austragsrohrs angeordnet ist, wobei am Zylinder zur Längsachse des Zylinders geneigte Flügel angeordnet sind und der Zylinder mit seinem Außengewinde in das Innengewinde des Hohlraums über eine Öffnung eingreift, die der Austragsöffnung des Austragsrohrs entgegen gesetzt ist.

Alternativ kann vorgesehen sein, dass der Zylinder im Austragsrohr oder in einem auf dem Kartuschenkopf befestigten Zylinder mit Stegen, die mit einem Zylinderende verbunden sind, fixiert ist, wobei der Zylinder mit dem freien Zylinderende entgegengesetzt zur Richtung der Austragsöffnung des Austragsrohrs angeordnet ist, der Hohlkörper mit seinem Innengewinde in das Außengewinde des Zylinders greift, eine Öffnung des Hohlkörpers in Richtung der Austragsöffnung des Austragsrohrs parallel zum Innengewinde des Hohlkörpers angeordnet ist und dass zur Längsachse des Hohlkörpers geneigte Flügel am Hohlkörper angebracht sind.

Ein erfindungsgemäßes Verfahren zum Austragen und Vermischen von Polymethylmethacrylat-Knochenzementpaste mit einer erfindungsgemäßen Mischvorrichtung kann dadurch realisiert werden, dass ein Kolben in einer Kartusche eine Polymethylmethacrylat-Knochenzementpaste in Richtung Kartuschenkopf presst, wobei die Polymethylmethacrylat-Knochenzementpaste die Vorrichtung ganz oder teilweise umfließt und dadurch Flügel an einem Hohlkörper oder an einem Zylinder (Förderkolben) beaufschlagt, so dass eine relative Drehbewegung des Hohlkörpers gegenüber des Zylinders bewirkt wird, wobei der Zylinder in den Hohlkörper durch den Eingriff seines Außengewindes in ein Innengewinde des Hohlkörpers gedreht wird, wobei das Volumen des Hohlraums im Hohlkörper proportional zu den Umdrehungen des Zylinders zum Hohlkörper vermindert wird und der im Hohlraum enthaltene Beschleuniger oder Initiator proportional zu den Umdrehungen und damit proportional zum ausgepressten Volumen der Zementpaste in die strömende Zementpaste gedrückt wird.

Im Folgenden werden Ausführungsbeispiele der Erfindung anhand von vier schematisch dargestellten Figuren erläutert, ohne jedoch dabei die Erfindung zu beschränken. Dabei zeigt:
- Figur 1:: eine schematische Querschnittansicht einer erfindungsgemäßen Mischvorrichtung;
- Figur 2:: eine schematische perspektivische Ansicht einer erfindungsgemäßen Mischvorrichtung mit transparentem Gehäuse;
- Figur 3:: eine schematische Querschnittansicht einer erfindungsgemäßen Mischvorrichtung mit angeschlossenem Austragsrohr und angeschlossener Kartusche; und
- Figur 4:: eine schematische Querschnittansicht der erfindungsgemäßen Mischvorrichtung nach Figur 3 entlang eines senkrechten Schnitts A-A.

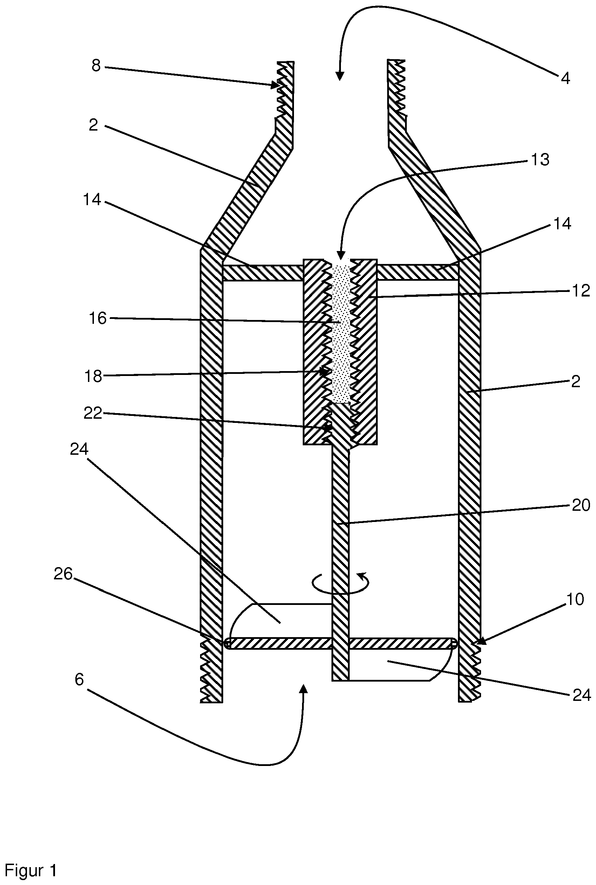

Figur 1 zeigt eine schematische Querschnittansicht einer erfindungsgemäßen Mischvorrichtung. Die Mischvorrichtung umfasst ein rotationssymmetrisches Gehäuse 2 aus einem thermoplastischen Kunststoff. Das Gehäuse 2 ist als zylindrisches Rohr aufgebaut, dass sich nach oben über einen konischen Abschnitt verjüngt und danach wieder zylindrisch geformt ist. An beiden Enden des Gehäuses 2 sind Öffnungen 4, 6 vorgesehen. Durch die obere Öffnung 4 kann das herzustellende Mehrkomponentengemisch (nicht gezeigt) herausgepresst werden, während durch die untere Öffnung eine erste Komponente (nicht gezeigt) in das Gehäuse 2 hereingepresst wird.

An beiden Enden des Gehäuses 2 sind Außengewinde 8, 10 vorgesehen. An dem oberen Gewinde 8 kann ein Austragsrohr (nicht gezeigt) mit einem statischen Mischer aufgeschraubt werden. An dem unteren Gewinde 10 wird eine Kartusche (nicht gezeigt) befestigt, die die erste Komponente enthält. Die Kartusche umfasst einen Kolben, mit dem die erste Komponente in das Gehäuse gepresst werden kann. Die Kartusche und das Austragsrohr können ebenfalls aus Kunststoff gefertigt sein.

Im Inneren des Gehäuses 2 ist ein zylindrischer Hohlkörper 12 angeordnet, der über zwei oder mehr Stege 14 in der Mitte des Gehäuses 2 angeordnet ist. Vorliegend ist der Hohlkörper 12 möglichst auf der Symmetrieachse des unteren zylindrischen Bereichs des Gehäuses 2 angeordnet. Im Inneren des Hohlkörpers 12 ist ein Hohlraum vorgesehen, der mit einer zweiten Komponente 16 gefüllt ist und der an beiden Deckflächen des zylindrischen Hohlkörpers 12 geöffnet ist. Durch die obere Öffnung 13 des Hohlkörpers 12 kann die zweite Komponente 16 an die Umgebung des Hohlkörpers 12 abgegeben werden. Die Innenwand des Hohlraums wird durch ein Innengewinde 18 gebildet.

Die untere Öffnung des Hohlkörpers 12 ist durch einen Förderkolben 20 verschlossen. Der Förderkolben 20 weist ein Außengewinde 22 auf, das in das Innengewinde 18 des Hohlkörpers 12 greift, wodurch die untere Öffnung des Hohlraums abgeschlossen ist. Der Förderkolben 20 kann also in den Hohlraum des Hohlkörpers 12 hineingeschraubt werden.

Am unteren Ende des Förderkolbens 20, der großenteils durch eine zylindrische Stange gebildet wird, die als Gewindestange endet, ist ein Vortriebselement 24 angeordnet, das fest mit dem Förderkolben 20 verbunden ist und vorzugsweise einteilig mit dem Förderkolben 20 ausgeführt sein kann. Das Vortriebselement 24 wird durch mehrere Flügel 24 gebildet, die bezogen auf die Drehachse (Symmetrieachse) des Förderkolbens 20, wie auch des Gehäuses 2 und des Hohlkörpers 12, alle gleich orientiert sind. Die Flügel 24 stehen schräg bezogen auf die untere Öffnung 6 und damit bezogen auf die Strömungsrichtung der ersten Komponente, wenn diese in das Gehäuse 2 gepresst wird. Das Vortriebselement 24 ist also wie ein Propeller oder eine Schiffsschraube oder genauer wie Windradflügel oder eine Turbine aufgebaut.

Durch eine von unten in das Gehäuse 2 einströmendes Fluid (die erste Komponente) wird das Vortriebselement 24 gedreht, wie durch den Pfeil angedeutet. Durch die Drehung wird der Förderkolben 20 in den Hohlraum des Hohlkörpers 12 hereingedreht und die zweite Komponente 16 aus dem Hohlkörper 12 in den Fluidstrom der ersten Komponente herausgepresst. Dort vermischt sich die erste Komponente mit der zweiten Komponente 16 und es wird ein Zwei-Komponenten-Gemisch erzeugt, das über die obere Öffnung 4 aus dem Gehäuse 2 herausgedrückt wird.

Um dem Vortriebselement 24 Spiel zu lassen aber dennoch eine Position vorzugeben, sind an den äußersten Spitzen der Flügel 24 Kontaktpunkte 26 in Form von Halbkugeln vorgesehen, die einen punktuellen Kontakt jedes Flügels 24 mit der zylindrischen Innenwand des Gehäuses 2 sicherstellen und so den Förderkolben 20 und das Vortriebselement positionieren.

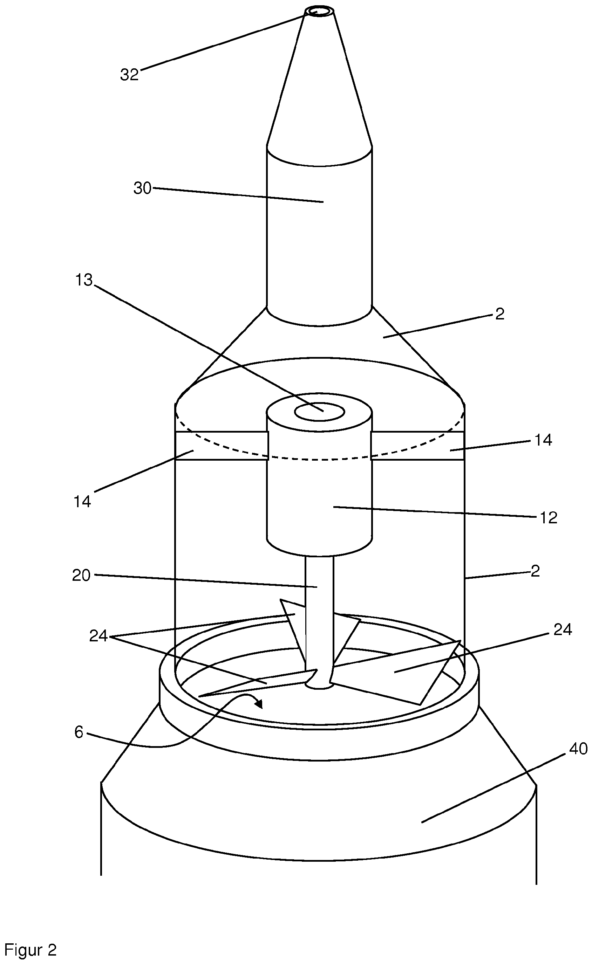

Figur 2 zeigt eine schematische perspektivische Ansicht einer alternativen erfindungsgemäßen Mischvorrichtung mit einem Gehäuse 2, bei dem die vordere, dem Betrachter zugewandte Wandung des Gehäuses 2 transparent gezeigt ist, um einen Blick ins Innere der Mischvorrichtung zu ermöglichen. Die vordere Kante der Wandung des Gehäuses 2 ist durch eine gestrichelte Linie angedeutet. Das Gehäuse 2 hat einen konischen Bereich (in Figur 2 oben) und einen zylindrischen Bereich (in Figur 2 unten).

Im Inneren des Gehäuses 2 ist ein zylindrischer Hohlkörper 12 mit einer oberen Öffnung 13 angeordnet. Zwei Stege 14 halten den Hohlkörper 12 mittig in dem Gehäuse 2. Die Innenwandung des zylindrischen Hohlkörpers 12 ist durch ein Innengewinde (nicht zu sehen) gebildet. In dieses Innengewinde ist ein Förderkolben 20 in Form einer Stange mit einem Außengewinde am oberen Ende teilweise eingeschraubt. Die Gewinde sind hier als Linksgewinde ausgeführt, es ist aber ohne weiteres möglich Rechtsgewinde zu verwenden. Am unteren Ende des Förderkolbens 20 ist ein Vortriebselement 24 mit drei geneigten Propellerflügeln 24 angeordnet. Wenn Rechtsgewinde für den Hohlkörper 12 und den Förderkolben 20 verwendet werden sollen, muss die Neigung der Flügel 24 umgekehrt werden.

An der Oberseite des Gehäuses 2 ist ein Austragsrohr 30 aufgeschraubt. An der Spitze des Austragsrohrs 30 ist eine Öffnung 32 vorgesehen, durch die das fertige Zwei-Komponenten-Gemisch appliziert werden kann. Im Inneren des Austragsrohrs 30 kann ein statischer Mischer zum besseren Durchmischen des Zwei-Komponenten-Gemischs vorgesehen sein.

Am unteren Ende des Gehäuses 2 ist eine Kartusche 40 befestigt, die die erste Komponente beinhaltet. Das Gehäuse 2 ist nach oben und unten geöffnet. Die untere Öffnung 6 ist unterhalb des Vortriebselements 24 zu erkennen und erlaubt einen Blick in die Kartusche 40, während die obere Öffnung durch das Austragsrohr 30 verdeckt ist. Die Kartusche 40 ist dazu ausgelegt, dass die in ihr enthaltene erste Komponente durch die Öffnung 6 in das Gehäuse 2 gepresst werden kann. Dadurch wird der Propeller 24 gedreht und der Förderkolben 20 schraubt sich in den Hohlkörper 12. Dadurch wird der Inhalt des Hohlkörpers 12, das heißt die zweite Komponente in den Strom der ersten Komponente gedrückt, die den Hohlkörper 12 umfließt.

Das Gemisch wird anschließend in dem Austragsrohr 30 mit dem statischen Mischer stärker gemischt und kann schließlich durch die Öffnung 32 in der Austragsrohrspitze appliziert werden.

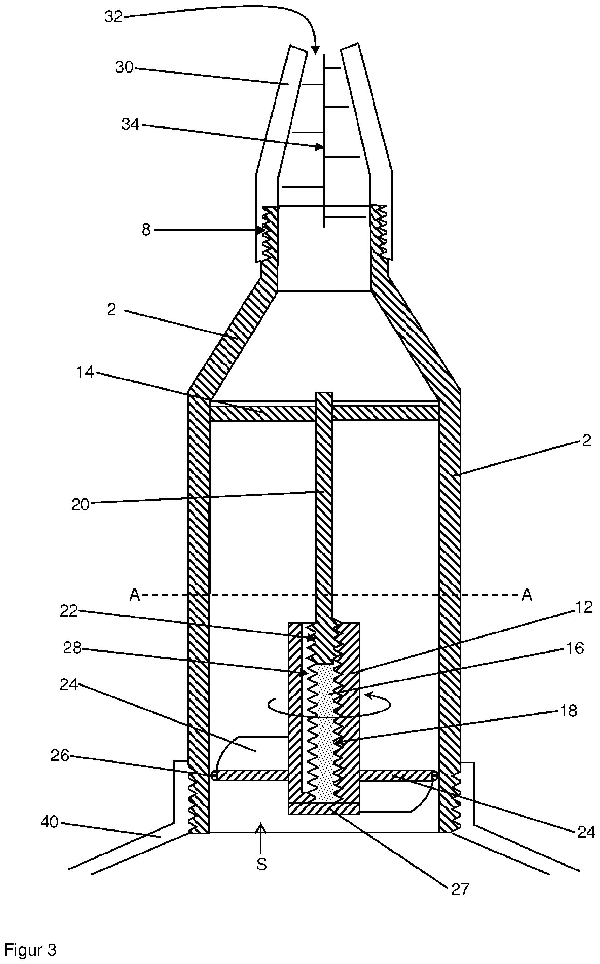

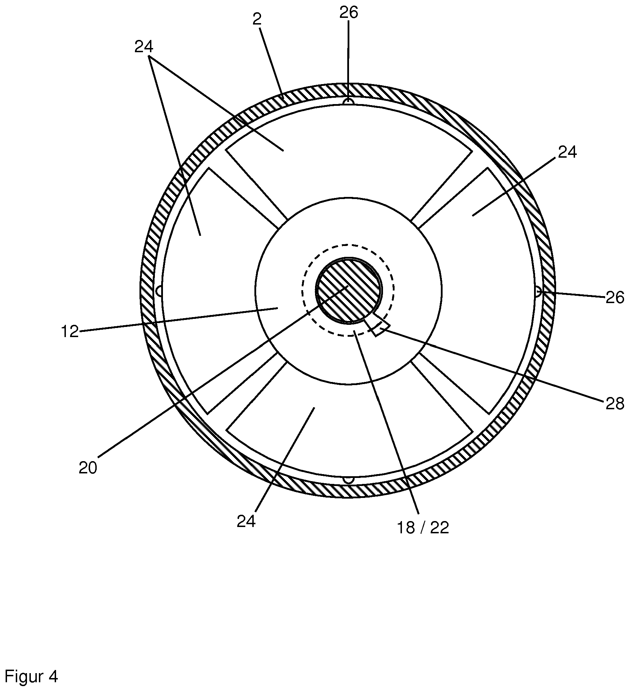

Figur 3 zeigt eine schematische Querschnittansicht einer weiteren alternativen erfindungsgemäßen Mischvorrichtung mit angeschlossenem Austragsrohr 30 und angeschlossener Kartusche 40. Dabei zeigt Figur 4 eine schematische Querschnittansicht der erfindungsgemäßen Mischvorrichtung nach Figur 3 entlang eines senkrechten Schnitts A-A, der in Figur 3 als gestrichelte Linie dargestellt ist.

Diese Ausführungsform ist ähnlich den Ausführungsformen nach den Figuren 1 oder 2 aufgebaut. Der Hauptunterschied besteht darin, dass hier nicht der Hohlkörper 12 sondern ein Förderkolben 20 durch Stege 14 fest mit dem Gehäuse 2 verbunden ist. Das Vortriebselement 24 ist dementsprechend nicht mit dem Förderkolben 20 sondern mit dem Hohlkörper 12 fest verbunden. Der Förderkolben 20 ist ein Zylinder aus Kunststoff mit einem Außengewinde 22, das sich in ein Innengewinde 18 des Hohlkörpers 12 dreht. Im Unterschied zu den in den Figuren 1 und 2 beschriebenen Ausführungsformen der Erfindung wird also hier nicht der Förderkolben 20 durch das Vortriebselement 24 angetrieben und in Drehung versetzt, sondern der Hohlkörper 12 wird durch das Vortriebelement 24 angetrieben und in Drehung versetzt. In beiden Fällen wird der Förderkolben 20 in den Hohlraum des Hohlkörpers 12 geschraubt.

Ein weiterer Unterschied in dem in den Figuren 3 und 4 gezeigten Aufbau der Mischvorrichtung zu denen nach den Figuren 1 und 2 ist, dass die in dem Hohlkörper 12 enthaltene zweite Komponente 16 nicht durch die Öffnung herausgedrückt wird, die dem Förderkolben 20 gegenüberliegt, da diese Öffnung hier durch eine Platte 27 verschlossen ist. Stattdessen ist Längsnut 28 im Inneren des Hohlkörpers 12 vorgesehen, durch die die zweite Komponente 16 in Richtung des Förderkolbens 20 aus dem Hohlkörper 12 gepresst wird. Dieser Aufbau dient dazu, dass die zweite Komponente 16 in die Strömungsrichtung der ersten Komponente, die in Figur 3 durch den Pfeil S angedeutet ist, aus der Kartusche 40 gepresst wird.

Dort vermischt sich die zweite Komponente 16 aus dem Hohlkörper 12 mit der ersten Komponente aus der Kartusche 40. Wie auch bei den Ausführungsbeispielen nach den Figuren 1 und 2 wird das Gemisch anschließend an den Stegen 14 vorbei durch eine vordere Öffnung des Gehäuses 2 ausgepresst. Auf dem Außengewinde 8 an der vorderen Öffnung des Gehäuses 2 ist ein Austragsrohr 30 aufgeschraubt und befestigt, das einen statischen Mischer 34 enthält. Das Gemisch wird durch den statischen Mischer 34 durchmischt, bevor es aus der Applikationsöffnung 32 an der Austragsrohrspitze ausgetragen wird.

Statt nur ein Zwei-Komponenten-Gemisch zu erzeugen, können erfindungsgemäße Mischvorrichtungen ohne weiteres auch Mehrkomponentengemische aus mehreren Komponenten erzeugen. Beispielsweise kann die Kartusche 40 oder der Hohlkörper 12 mit mehr als einer Komponente gefüllt sein, die sich nicht gegenseitig stören. Ebenso können aber auch mehrere der gezeigten Hohlkörper 12 mit mehreren Förderkolben 20 und mehreren Vortriebselementen 24 in einem längeren Gehäuse 2 hintereinander oder alternativ dazu nebeneinander angeordnet sein.

### Bezugszeichenliste

- 2: Gehäuse
- 4: Vordere Öffnung
- 6: Hintere Öffnung
- 8, 10: Außengewinde
- 12: Hohlkörper
- 14: Steg
- 16: Zweite Komponente
- 18: Innengewinde
- 20: Förderkolben / Zylinderstange
- 22: Außengewinde
- 24: Vortriebselement / Flügel
- 26: Kontaktpunkt
- 27: Platte
- 28: Längsnut
- 30: Austragsrohr
- 32: Öffnung / Austragsrohröffnung
- 34: Statischer Mischer
- 40: Kartusche für erste Komponente
- A: Querschnittebene der Figur 4
- S: Fluidstrom / Fluidstromrichtung der ersten Komponente

## Patentansprüche

1. Mischvorrichtung zum Herstellen eines Mehrkomponentengemischs, insbesondere für medizinische Anwendungen, das zumindest eine erste Komponente und eine zweite Komponente (16) umfasst, wobei die erste Komponente ein fluide Masse, insbesondere eine pastöse Masse ist, wobei die Mischvorrichtung ein Gehäuse (2) mit wenigstens einer ersten Öffnung (4) und einen Hohlkörper (12) umfasst, wobei der Hohlkörper (12) einen Innenraum umfasst, der die zweite Komponente (16) beinhaltet und die Mischvorrichtung einen Förderkolben (20) zum Austreiben der zweiten Komponente (16) aus dem Innenraum des Hohlkörpers (12) umfasst, **dadurch gekennzeichnet, dass**
der Hohlkörper (12) ein Gewinde (18) aufweist, der Förderkolben (20) ein Gegengewinde (22) umfasst, das in das Gewinde (18) des Hohlkörpers (12) greift, und die Mischvorrichtung ein Vortriebselement (24) umfasst, das innerhalb des Gehäuses (2) angeordnet ist, wobei das Vortriebselement (24) eine Strömung (S) der ersten Komponente durch das Gehäuse (2) in eine Drehbewegung umsetzt, wobei die Drehbewegung des Vortriebselements (24) den Förderkolben (20) in den Innenraum des Hohlkörpers (12) schraubt und dadurch die zweite Komponente (16) aus dem Hohlraum in den Fluidstrom der ersten Komponente pressbar ist.

2. Mischvorrichtung nach Anspruch 1, **dadurch gekennzeichnet, dass**
das Gewinde (18) des Hohlkörpers (12) ein Innengewinde (18) ist und das Gegengewinde (22) des Förderkolbens (20) ein Außengewinde (22) ist, das in das Innengewinde (18) des Hohlkörpers (12) greift, wobei vorzugsweise die Innenwand des Innenraums durch das Innengewinde (18) gebildet wird.

3. Mischvorrichtung nach Anspruch 1 oder 2, **dadurch gekennzeichnet, dass**
der Hohlkörper (12), vorzugsweise über Stege (14) fest mit dem Gehäuse (2) verbunden ist und das Vortriebselement (24) fest mit dem Förderkolben (20) verbunden ist oder der Förderkolben (20), vorzugsweise über Stege (14) fest mit dem Gehäuse (2) verbunden ist und das Vortriebselement (24) fest mit dem Hohlkörper (12) verbunden ist.

4. Mischvorrichtung nach einem der vorangehenden Ansprüche, **dadurch gekennzeichnet, dass**
der Förderkolben (20) gegen den Hohlkörper (12) drehbar gelagert ist und in Längsrichtung des Innenraums des Hohlkörpers (12) schraubbar gelagert ist.

5. Mischvorrichtung nach einem der vorangehenden Ansprüche, **dadurch gekennzeichnet, dass**
der Innenraum des Hohlkörpers (12) zwei Öffnungen aufweist, wobei eine Öffnung durch den Förderkolben (20) verschlossen ist und bevorzugt die andere Öffnung (13) in Richtung des Fluidstroms (S) der ersten Komponente geöffnet ist, wobei besonders bevorzugt der Innenraum des Hohlkörpers (12) im Wesentlichen zylindrisch geformt ist und die beiden Öffnungen an den Grundflächen des zylindrischen Innenraums angeordnet sind.

6. Mischvorrichtung nach einem der vorangehenden Ansprüche, **dadurch gekennzeichnet, dass**
das Vortriebselement (24) eine Förderschraube ist oder zumindest zwei Flügel (24) und/oder eine Flügelscheibe umfasst, wobei die Flügel (24) und/oder die Flügelscheibe zumindest eine gegen den Fluidstrom (S) der ersten Komponente geneigte Fläche umfasst oder umfassen oder vorzugsweise mehrere gegen den Fluidstrom (S) der ersten Komponente geneigte Flächen umfasst oder umfassen.

7. Mischvorrichtung nach Anspruch 6, **dadurch gekennzeichnet, dass**
die geneigte Fläche oder die geneigten Flächen der Flügel (24) und/oder Flügelscheibe zwischen 5° und 85° gegen den Fluidstrom (S) der ersten Komponente geneigt ist oder sind, vorzugsweise zwischen 30° und 60°, besonders bevorzugt um 45°.

8. Mischvorrichtung nach einem der vorangehenden Ansprüche, **dadurch gekennzeichnet, dass**
an dem Vortriebselement (24) bezogen auf die Drehachse des Vortriebselements (24) außen liegend Kontaktpunkte (26) vorgesehen sind, über die das Vortriebselement (24) im Inneren des Gehäuses (2) führbar sind, wobei das Gehäuse (2) zumindest in diesem Bereich innen zylindrisch ist.

9. Mischvorrichtung nach einem der vorangehenden Ansprüche, **dadurch gekennzeichnet, dass**
das Mehrkomponentengemisch ein Zweikomponentengemisch ist, insbesondere ein aushärtender medizinischer Zement, wobei eine Komponente einen Beschleuniger umfasst und die andere Komponente einen Initiator umfasst, wobei bevorzugt die erste Komponente Methacrylat-Monomere und darin gelöste Polymere umfasst und besonders bevorzugt eine Polymethylmethacrylat-Knochenzementpaste ist und die zweite Komponente (16) einen den Initiator, insbesondere ein radikalisches Initiatorsystem umfasst.

10. Mischvorrichtung nach einem der vorangehenden Ansprüche, **dadurch gekennzeichnet, dass**
an das Gehäuse (2) eine Kartusche (40) anschließbar ist oder befestigt ist, vorzugsweise an einer zweiten Öffnung (6) des Gehäuses (2), wobei die Kartusche (40) die erste Komponente enthält und die Kartusche (40) einen Kolben zum Austreiben der ersten Komponente aus der Kartusche (40) und zum Erzeugen des Fluidstroms (S) der ersten Komponente durch das Gehäuse (2) umfasst, wobei bevorzugt die Kartusche (40) über ein Befestigungsmittel (10) mit dem Gehäuse (2) verbindbar oder verbunden ist, besonders bevorzugt über eine Gewinde (10), einen Bajonett-Verschluss oder eine Rastung.

11. Mischvorrichtung nach einem der vorangehenden Ansprüche, **dadurch gekennzeichnet, dass**
an das Gehäuse (2) ein Austragsrohr (30) als Fortsetzung der wenigstens einen ersten Öffnung (4) anschließbar ist oder befestigt ist, so dass das Mehrkomponentengemisch über das Austragsrohr (30) austragbar ist, wobei bevorzugt im Inneren des Austragsrohrs (30) ein statischer Mischer (34) zum Durchmischen des Mehrkomponentengemischs angeordnet ist.

12. Mischvorrichtung nach einem der vorangehenden Ansprüche, **dadurch gekennzeichnet, dass**
die zweite Komponente (16) ein Feststoff ist, der in dem Innenraum des Hohlkörpers (12) steckt und der sich in dem Fluidstrom (S) der ersten Komponente löst.

13. Mischvorrichtung nach einem der vorangehenden Ansprüche, **dadurch gekennzeichnet, dass**
der Hohlkörper (12) zumindest bereichsweise innerhalb des Gehäuses (2) angeordnet ist, vorzugsweise der Hohlkörper (12) und der Förderkolben (20) vollständig im Inneren des Gehäuses (2) angeordnet sind, so dass beide von dem Fluidstrom (S) der ersten Komponente umfließbar sind.

14. Mischvorrichtung nach einem der vorangehenden Ansprüche, **dadurch gekennzeichnet, dass**
beim Einschrauben des Förderkolbens (20) in den Innenraum des Hohlkörpers (12) sich das Volumen des Innenraums des Hohlkörpers (12) proportional zu der Umdrehung des Vortriebselements (24) und damit proportional zu der Umdrehung des Förderkolbens (20) gegen den Hohlkörper (12) vermindert und die Umdrehungsgeschwindigkeit des Förderkolbens (20) proportional zum Volumenstrom der ersten Komponente durch das Vortriebselement (24) ist.

15. Kartuschensystem umfassend eine Mischvorrichtung nach einem der vorangehenden Ansprüche, eine Kartusche (40), die die erste Komponente beinhaltet und die einen Kolben zum Ausdrücken der ersten Komponente in die Mischvorrichtung umfasst.

16. Applikator umfassend ein Kartuschesystem nach Anspruch 15, ein bedienbares Ventil und eine Druckgaspatrone, insbesondere eine CO₂-Patrone zur Beaufschlagung des Kolbens der Kartusche (40) mit einem Gasdruck.

17. Verfahren zum Herstellen eines Mehrkomponentengemischs, insbesondere mit einer Mischvorrichtung nach einem der Ansprüche 1 bis 14, wobei eine erste Komponente in ein Gehäuse (2) gepresst wird und dort einen Fluidstrom (S) erzeugt, und der Fluidstrom (S) ein Vortriebselement (24) antreibt, **dadurch gekennzeichnet, dass** der Fluidstrom (S) das Vortriebselement (24) in Drehung versetzt und durch das sich drehende Vortriebselement (24) ein Förderkolben (20) in einen Innenraum eines Hohlkörpers (12) geschraubt wird, wobei dadurch eine zweite Komponente (16) aus dem Inneren des Hohlköpers (12) in den Fluidstrom (S) der ersten Komponente gedrückt wird und sich dort mit der ersten Komponente mischt und das Gemisch aus dem Gehäuse (2) heraus gedrückt wird.

18. Verfahren nach Anspruch 17, **dadurch gekennzeichnet, dass** beim Einschrauben des Förderkolbens (20) in den Innenraum des Hohlkörpers (12) das Volumen des Innenraums des Hohlkörpers (12) proportional zu der Umdrehung des Vortriebselements (24) und damit des Förderkolbens (20) gegen den Hohlkörper (12) vermindert wird und eine Umdrehungsgeschwindigkeit des Förderkolbens (20) proportional zum Volumenstrom (S) der ersten Komponente durch das Vortriebselement (24) erzeugt wird.

## Claims

1. A mixing device for producing a multi-component mixture, in particular for medical applications, the mixture comprising at least one first component and one second component (16), wherein the first component is a fluid mass, in particular a pasty mass, wherein the mixing device comprises a housing (2) with at least one first opening (4) and a hollow body (12), wherein the hollow body (12) comprises an internal space containing the second component (16), and the mixing device comprises a delivery plunger (20) for driving the second component (16) out of the internal space of the hollow body (12), **characterised in that**
the hollow body (12) has a thread (18), the delivery plunger (20) comprises a mating thread (22) engaging the thread (18) of the hollow body (12), and the mixing device comprises a propulsive element (24) arranged inside the housing (2), wherein the propulsive element (24) converts a flow (S) of the first component through the housing (2) into a rotary movement, wherein the rotary movement of the propulsive element (24) screws the delivery plunger (20) into the internal space of the hollow body (12) and the second component (16) can thereby be pressed out of the hollow space into the fluid flow of the first component.

2. The mixing device in accordance with Claim 1, **characterised in that**
the thread (18) of the hollow body (12) is an internal thread (18) and the mating thread (22) of the delivery plunger (20) is an external thread (22) which engages the internal thread (18) of the hollow body (12), wherein, preferably, the inside wall of the internal space is formed by the internal thread (18).

3. The mixing device in accordance with Claims 1 or 2, **characterised in that**
the hollow body (12), preferably via webs (14), is securely connected to the housing (2) and the propulsive element (24) is securely connected to the delivery plunger (20) or the delivery plunger (20), preferably via webs (14), is securely connected to the housing (2) and the propulsive element (24) is securely connected to the hollow body (12).

4. The mixing device in accordance with any one of the preceding claims, **characterised in that**
the delivery plunger (20) is mounted against the hollow body (12) in a rotary manner and mounted in the longitudinal direction of the internal space of the hollow body (12) in a screwable manner.

5. The mixing device in accordance with any one of the preceding claims, **characterised in that**
the internal space of the hollow body (12) has two openings, wherein one opening is closed by the delivery plunger (20) and, preferably, the other opening (13) is open in the direction of the fluid flow (S) of the first component, wherein, most preferably, the internal space of the hollow body (12) is, in essence, shaped cylindrically and the two openings are arranged on the base areas of the cylindrical internal space.

6. The mixing device in accordance with any one of the preceding claims, **characterised in that**
the propulsive element (24) is a conveyor screw or comprises at least two blades (24) and/or a blade disc, wherein the blades (24) and/or the blade disc comprises or comprise at least one surface that is inclined against the fluid flow (S) of the first component or preferably comprises or comprise a plurality of surfaces that are inclined against the fluid flow (S) of the first component.

7. The mixing device in accordance with Claim 6, **characterised in that**
the inclined surface or the inclined surfaces of the blades (24) and/or the blade disc is or are inclined against the fluid flow (S) of the first component by between 5° and 85°, preferably between 30° and 60°, most preferably by about 45°.

8. The mixing device in accordance with any one of the preceding claims, **characterised in that**
contact points (26) are provided on the outside of the propulsive element (24) in relation to the axis of rotation of the propulsive element (24), with the propulsive element (24) being guidable in the interior region of the housing (2) via said contact points (26), wherein the housing (2) is cylindrical inside, at least in this region.

9. The mixing device in accordance with any one of the preceding claims, **characterised in that**
the multi-component mixture is a two-component mixture, in particular a curing medical cement, wherein one component comprises an accelerator and the other component comprises an initiator, wherein, preferably, the first component comprises methacrylate monomers and polymers dissolved therein and, most preferably, is a bone cement paste consisting of polymethyl methacrylate, and the second component (16) comprises the initiator, in particular a radical initiator system.

10. The mixing device in accordance with any one of the preceding claims, **characterised in that**
a cartridge (40) is connectable or fixed to the housing (2), preferably at a second opening (6) of the housing (2), wherein the cartridge (40) contains the first component and the cartridge (40) comprises a plunger for driving the first component out of the cartridge (40) and for producing the fluid flow (S) of the first component through the housing (2), wherein, preferably, the cartridge (40) is connectable or connected to the housing (2) via a fixing means, most preferably via a thread (10), a bayonet catch or a detent.

11. The mixing device in accordance with any one of the preceding claims, **characterised in that**
a discharge tube (30) is connectable or fixed to the housing (2) as a continuation of the at least one first opening (4), with the result that the multi-component mixture can be discharged through the discharge tube (30), wherein, preferably, a static mixer (34) is arranged in the interior region of the discharge tube (30) for stirring the multi-component mixture.

12. The mixing device in accordance with any one of the preceding claims, **characterised in that**
the second component (16) is a solid which is inserted in the internal space of the hollow body (12) and which dissolves in the fluid flow (S) of the first component.

13. The mixing device in accordance with any one of the preceding claims, **characterised in that**
the hollow body (12) is arranged inside the housing (2) at least in certain regions and, preferably, the hollow body (12) and the delivery plunger (20) are completely arranged in the interior region of the housing (2), with the result that both can be flowed around by the fluid flow (S) of the first component.

14. The mixing device in accordance with any one of the preceding claims, **characterised in that**
when the delivery plunger (20) is screwed into the internal space of the hollow body (12), the volume of the internal space of the hollow body (12) decreases proportionately to the rotation of the propulsive element (24) and thus proportionately to the rotation of the delivery plunger (20) against the hollow body (12) and that the speed of rotation of the delivery plunger (20) is proportional to the volume flow of the first component through the propulsive element (24).

15. A cartridge system, comprising a mixing device in accordance with any one of the preceding claims, and a cartridge (40) which contains the first component and comprises a plunger for pressing the first component into the mixing device.

16. An applicator, comprising a cartridge system in accordance with Claim 15, an operable valve and a compressed gas cartridge, in particular a CO₂ cartridge for applying a gas pressure to the plunger of the cartridge (40).

17. A method for producing a multi-component mixture, in particular with a mixing device in accordance with any one of Claims 1 to 14, wherein a first component is pressed into a housing (2) and produces a fluid flow (S) there, and wherein the fluid flow (S) drives a propulsive element (24), **characterised in that** the fluid flow (S) puts the propulsive element (24) into rotation and a delivery plunger (20) is screwed into an internal space of a hollow body (12) through said rotating propulsive element (24), wherein a second component (16) is thereby pressed from the interior region of the hollow body (12) into the fluid flow (S) of the first component and mixes with the first component there, and that the mixture is pressed out of the housing (2).

18. The method in accordance with Claim 17, **characterised in that**
when the delivery plunger (20) is screwed into the internal space of the hollow body (12), the volume of the internal space of the hollow body (12) is decreased proportionately to the rotation of the propulsive element (24) and thus of the delivery plunger (20) against the hollow body (12) and that the speed of rotation of the delivery plunger (20) is produced proportionately to the volume flow (S) of the first component through the propulsive element (24).

## Revendications

1. Dispositif mélangeur pour la fabrication d'un mélange multi-composants, en particulier pour les applications médicales, comprenant au moins un premier composant et un deuxième composant (16), le premier composant étant une masse fluide, en particulier une masse pâteuse, le dispositif mélangeur comprenant un boîtier (2) avec au moins une première ouverture (4) et un corps creux (12), le corps creux (12) comprenant un espace intérieur contenant le deuxième composant (16) et le dispositif mélangeur comprenant un piston d'alimentation (20) pour l'expulsion du deuxième composant (16) de l'espace intérieur du corps creux (12), **caractérisé par le fait que** le corps creux (12) présente un filetage (18), le piston d'alimentation (20) comprend un contre-filetage (22) qui entre dans le filetage (18) du corps creux (12) et le dispositif mélangeur comprend un élément moteur (24) qui est disposé à l'intérieur du boîtier (2), l'élément moteur (24) appliquant dans un mouvement de rotation un flux (S) du premier composant par le boîtier (2), le mouvement de rotation de l'élément moteur (24) vissant le piston d'alimentation (20) dans l'espace intérieur du corps creux (12) et le deuxième composant (16) pouvant, de ce fait, être extrait de l'interstice dans le flux de fluide du premier composant.

2. Dispositif mélangeur conformément à la revendication n°1, **caractérisé par le fait que** le filetage (18) du corps creux (12) est un filetage intérieur (18) et que le contre-filetage (22) du piston d'alimentation (20) est un filetage extérieur (22) qui entre dans le filetage intérieur (18) du corps creux (12), la paroi intérieure de l'espace intérieur étant de préférence constituée par le filetage intérieur (18).

3. Dispositif mélangeur conformément à la revendication n°1 ou n°2, **caractérisé par le fait que** le corps creux (12) est relié de façon fixe au boîtier (2), de préférence par des entretoises (14), et que l'élément moteur (24) est relié de façon fixe au piston d'alimentation (20) ou que le piston d'alimentation (20) est relié de façon fixe au boîtier (2), de préférence par des entretoises (14), et que l'élément moteur (24) est relié de façon fixe au corps creux (12).

4. Dispositif mélangeur conformément à l'une des revendications précédentes, **caractérisé par le fait que** le piston d'alimentation (20) est monté de façon à pouvoir tourner contre le corps creux (12) et monté de façon à pouvoir être vissé dans le sens longitudinal de l'espace intérieur du corps creux (12).

5. Dispositif mélangeur conformément à l'une des revendications précédentes, **caractérisé par le fait que** l'espace intérieur du corps creux (12) présente deux ouvertures, une ouverture étant obturée par le piston d'alimentation (20) et, de préférence, l'autre ouverture (13) étant ouverte en direction du flux de fluide (S) du premier composant, plus préférablement l'espace intérieur du corps creux (12) étant essentiellement formé de façon cylindrique et les deux ouvertures étant disposées sur les surfaces de base de l'espace intérieur cylindrique.

6. Dispositif mélangeur conformément à l'une des revendications précédentes, **caractérisé par le fait que** l'élément moteur (24) est une hélice transporteuse ou comprend au moins deux ailes (24) et/ou un disque aileté, les ailes (24) et/ou le disque aileté comprenant au moins une surface inclinée par rapport au flux de fluide (S) du premier composant ou, de préférence, comprenant plusieurs surfaces inclinées par rapport au flux de fluide (S) du premier composant.

7. Dispositif mélangeur conformément à la revendication n°6, **caractérisé par le fait que** la surface inclinée ou les surfaces inclinées des ailes (24) et/ou du disque aileté est inclinée ou sont inclinées entre 5° et 85° par rapport au flux de fluide (S) du premier composant, de préférence entre 30° et 60°, plus préférablement à 45°.

8. Dispositif mélangeur conformément à l'une des revendications précédentes, **caractérisé par le fait que** sur l'élément moteur (24) par rapport à l'axe pivotant de l'élément moteur (24) sont prévus des points de contact (26) situés à l'extérieur, par lesquels l'élément moteur (24) peut être guidé à l'intérieur du boîtier (2), le boîtier (2) étant cylindrique à l'intérieur au moins dans cette zone.

9. Dispositif mélangeur conformément à l'une des revendications précédentes, **caractérisé par le fait que** le mélange multi-composants est un mélange bicomposant, en particulier un ciment médical durcissant, un composant comprenant un accélérateur et l'autre composant comprenant un amorceur, de préférence le premier composant comprenant des monomères de méthacrylate et des polymères dissous à l'intérieur et, plus préférablement, étant une pâte de ciment osseux à base de polyméthacrylate de méthyle, et le deuxième composant (16) comprenant un amorceur, en particulier un système amorceur radicalaire

10. Dispositif mélangeur conformément à l'une des revendications précédentes, **caractérisé par le fait que** une cartouche (40) est reliable ou fixée au boîtier (2), de préférence à une deuxième ouverture (6) du boîtier (2), la cartouche (40) contenant le premier composant et la cartouche (40) comprenant un piston pour l'expulsion du premier composant de la cartouche (40) et pour la génération du flux de fluide (S) du premier composant par le boîtier (2), la cartouche (40) étant de préférence reliable ou reliée au boîtier (2) par un moyen de fixation (10), plus préférablement par un filetage (10), un verrouillage par baïonnette ou un cran.

11. Dispositif mélangeur conformément à l'une des revendications précédentes, **caractérisé par le fait que** un tuyau de sortie (30) est reliable ou fixé sur le boîtier (2) comme continuation au moins de la première ouverture (4) de façon à ce que le mélange multi-composants puisse être évacué par le tuyau de sortie (30), un mélangeur statique (34) étant de préférence disposé à l'intérieur du tuyau de sortie (30) pour mélanger le mélange multi-composants.

12. Dispositif mélangeur conformément à l'une des revendications précédentes, **caractérisé par le fait que** le deuxième composant (16) est un solide qui se trouve dans l'espace intérieur du corps creux (12) et qui se dissout dans le flux de fluide (S) du premier composant.

13. Dispositif mélangeur conformément à l'une des revendications précédentes, **caractérisé par le fait que** le corps creux (12) est disposé au moins partiellement à l'intérieur du boîtier (2), le corps creux (12) et le piston d'alimentation (20) sont de préférence entièrement disposés à l'intérieur du boîtier (2) de façon à ce que les deux puissent être entourés du flux de fluide (S) du premier composant.

14. Dispositif mélangeur conformément à l'une des revendications précédentes, **caractérisé par le fait que** le volume de l'espace intérieur du corps creux (12) diminue proportionnellement au nombre de tours de l'élément moteur (24) et, par conséquent, proportionnellement au nombre de tours du piston d'alimentation (20) par rapport au corps creux (12) lors du vissage du piston d'alimentation (20) dans l'espace intérieur du corps creux (12) et que la vitesse de rotation du piston d'alimentation (20) est proportionnelle au débit volumique du premier composant par l'élément moteur (24).

15. Système de cartouche comprenant un dispositif mélangeur conformément à l'une des revendications précédentes, une cartouche (40) qui contient le premier composant et qui comprend un piston pour l'expulsion du premier composant dans le dispositif mélangeur.

16. Applicateur comprenant un système de cartouche conformément à la revendication n°15, une vanne commandable et une cartouche de gaz comprimé, en particulier une cartouche de CO2 pour l'alimentation du piston de la cartouche (40) en pression de gaz.

17. Procédé pour la fabrication d'un mélange multi-composants, en particulier avec un dispositif mélangeur conformément à l'une des revendications n°1 à n° 14, un premier composant étant comprimé dans un boîtier (2) et y générant un flux de fluide (S), le flux de fluide (S) propulsant un élément moteur (24), **caractérisé par le fait que** le flux de fluide (S) met en rotation l'élément moteur (24) et que, par l'élément moteur (24) en rotation, un piston d'alimentation (20) est vissé dans un espace intérieur d'un corps creux (12), un deuxième composant (16) étant, de ce fait, expulsé de l'intérieur du corps creux (12) dans le flux de fluide (S) du premier composant, s'y mélangeant avec le premier composant et le mélange étant expulsé du boîtier (2).

18. Procédé conformément à la revendication n°17, **caractérisé par le fait que** le volume de l'espace intérieur du corps creux (12) diminue proportionnellement au nombre de tours de l'élément moteur (24) et, par conséquent, du piston d'alimentation (20) par rapport au corps creux (12) lors du vissage du piston d'alimentation (20) dans l'espace intérieur du corps creux (12) et qu'une vitesse de rotation du piston d'alimentation (20) est générée proportionnellement au débit volumique (S) du premier composant par l'élément moteur (24).
